# EUROPEAN PATENT SPECIFICATION

(11) **EP 1 429 724 B1**
(45) Date of publication and mention of the grant of the patent: **06.11.2013**
(21) Application number: 02766426.7
(22) Date of filing: 28.09.2002
(51) Int. Cl.: A61K 9/00, A61K 9/20, A23G 3/00, A23G 3/04, A61J 3/06, A61J 3/10, A61K 9/28, B30B 11/08, B30B 11/34, B30B 15/30, A23G 3/36, A23L 1/09, A61J 3/00, A23G 3/54, A23G 1/54

(54) **DOSAGE FORM CONTAINING A CONFECTIONERY COMPOSITION**
DARREICHUNGSFORM EINE SÜSSWARENZUBEREITUNG ENTHALTEND
FORME DE DOSAGE CONTENANT UNE COMPOSITION DE CONFISERIE

(30) Priority: 28.09.2001 US 966939; 28.09.2001 US 966509; 28.09.2001 US 966497; 28.09.2001 US 967414; 28.09.2001 US 966450
(43) Date of publication of application: 23.06.2004
(73) Proprietor: McNeil-PPC, Inc., Skillman, NJ 08558 (US)
(72) Inventor: BUNICK, Frank, J., Quakertown, PA 18951 (US); GILMOR, Timothy, P, Lansdale, PA 19446 (US); LABELLA, Gus, B, Collegeville, PA 19426 (US); MCNALLY, Gerard, P, Berwyn, PA 19312 (US); THOMAS, Martin, Lake Worth, FL 33467 (US); SOWDEN, Harry, S., Glenside, PA 19038 (US); LEE, Der-Yang, Flemington, NJ 08822 (US)
(74) Representative: Fisher, Adrian John
(86) International application number: PCT/US2002/031115
(87) International publication number: WO 2003/026614

(56) References cited:
- EP-A- 0 455 599
- EP-A- 0 572 731
- EP-A- 0 950 402
- WO-A-95/02396
- WO-A-97/06695
- US-A- 3 085 942
- US-A- 4 139 627
- US-A- 4 372 942
- US-A- 4 749 575
- US-A- 4 762 719
- US-A- 4 929 446
- US-A- 4 980 169
- US-A- 5 002 970
- US-A- 5 614 207
- US-A- 5 711 961
- US-A- 5 871 781
- US-A- 6 103 257

## Description

### BACKGROUND OF THE INVENTION

### 1. Field of the Invention

This invention relates to dosage forms such as pharmaceutical compositions comprising a confectionery composition. More particularly, this invention relates to dosage forms containing at least one active ingredient and a confectionery composition such as fat, amorphous sugar glass, or fondant.

### 2. Background Information

An important objective in designing pharmaceutical drug delivery systems, especially for pediatric and geriatric applications, is to make palatable, good tasting, dosage forms which mask the unpleasant taste and/or texture of the active pharmaceutical ingredient, to improve patient compliance with the dosing regimen. Confectionery compositions offer great potential for achieving these desired product attributes; however their use in pharmaceutical applications has to date been quite limited.

The substantial differences in regulations governing the confectionery and pharmaceutical industries has led to both facilities and processes that are not directly transferable between the two. The pharmaceutical developer desiring to apply the benefits of confectionery compositions to drug delivery systems must overcome major challenges in the areas of pharmaceutical "good manufacturing practices" (GMP) and product quality standards.

Confectionery manufacturing facilities, for example, are not commonly designed to allow for the rigorous controls on raw material traceability required by the pharmaceutical industry, or equipped for avoiding cross-contamination while processing multiple drug active ingredients. A high-purity water source, closed air-handling system, and on-site analytical laboratory are critical for any pharmaceutical operation. Installing and validating these systems entails substantial cost and complexity.

Most current commercial confectionery processes are designed to meet the primary objectives of high-volume and low cost, while aesthetic properties, consistency, uniformity, precision of dose, and general appearance are secondary considerations. For pharmaceutical dosage forms, there is a need for relatively low-cost, high volume commercial scale processing methods which also reliably and repeatably produce a highly precise dose which is also a uniform, consistent, elegant looking product.

In typical current confectionery processes, for example, a high percentage of scrap may be generated during processing without compromising efficiency, because the unused portion of a batch can be recycled into a future batch. In pharmaceutical processes, it would not be considered GMP to routinely re-process waste, or co-mingle batches.

Pharmaceutical dosage forms must have minimal variation in the weight of individual dosage units to achieve a high degree of drug content uniformity. Since many confectionery processes were designed without the need for the high degree of precision and reproducibility required for pharmaceuticals, a high degree of variation is inherent in their design. Typical confectionery rope-forming operations, for example, result in high product weight variation due to entrained air, and variations in rope tensile strength, viscosity, and plasticity with temperature. Conventional confectionery cut and wrap operations result in high product weight variation due to variation in flow and thickness of the rope. Extruded confectionery compositions can vary in piece weight due to changes in flow rate, and pulsations in the center fill pumping operation which lead to variations in center fill content. Additionally high weight variation is inherent in confectionery depositing operations, which do not fill a fixed volume, so piece weight depends on the reproducibility of the metered dose pumping, which is subject to variability in viscosity, due to temperature and material factors.

Product aesthetics represent another area of discrepancy between the two industries. For example, typical confectionery depositing operations produce a product which can have high definition on only three sides, but necessarily has a "free-formed" surface on one side. In such products, the degree of surface gloss is higher on the free-formed surface than the molded sides. Pharmaceutical products typically require a uniform and consistent appearance implying a high degree of control in the manufacturing process. It is thus desirable to have a manufacturing process which can provide uniform high definition on all sides of the dosage form.

The products produced by current confectionery methods are not designed to withstand conventional packaging operations without a substantial level of chipping, cracking, deformation, or breakage, which would be unacceptable for pharmaceutical dosage forms. For example typical rope forming operations entrain air during the cooling and folding operations, and impart mechanical stress to the glass during the roping operation. These imperfections manifest as strain lines and air bubbles in the finished product, which result in higher product fragility, decreased product uniformity, and decreased visual appeal. In particular, the areas of strain can serve as nucleation sites for crystal formation, which decreases the shelf life of the product. Entrained air additionally results in weight variation, and detracts from overall consistency and visual elegance of the product.

Chocolate-coatings over confectionery cores are typically made by filling a cold mold to solidify the outer layer of chocolate, then pouring out the excess, then injecting with the center fill material, or alternatively by dipping the core material into molten chocolate to form the bottom, then enrobing the remainder of the piece by pouring molten chocolate over the top and sides, then dripping off the excess. Such operations are inherently high in weight variation because the coating level depends upon the viscosity of the chocolate which will vary with raw materials, temperature, humidity, etc. Thus these operations do not lend themselves to pharmaceutical dosage forms. A need exists for manufacturing processes which enable the reliable and repeatable formation of a precise and consistent level of chocolate coating over any type of center fill, to realize the benefits of these types of compositions for pharmaceutical applications.

It is one object of this invention to provide a dosage form comprising an active ingredient and a confectionery composition wherein the relative standard deviation of the weight of the dosage form is less than 1%, and the dosage form has at least one face.

It is another object of this invention to provide a dosage form comprising an active ingredient and a confectionery composition wherein the dosage form has at least one face, the dosage form does not have a free formed surface, and the dosage form has a mean polarized light transmission at the angle of maximum extinction which is not greater than the mean polarized light transmission of the dosage form at the angle of maximum transmission.

Other objects, features and advantages of this invention will be apparent to these skilled in the art from the detailed description of the invention provided herein.

### SUMMARY OF THE INVENTION

In one embodiment, the present invention provides a dosage form obtainable by injecting at least one active ingredient and an amorphous sugar glass component into a molding chamber and molding into a desired shape using a thermal setting molding method and/or a thermal cycle molding method, wherein the dosage form has at least one face, and the relative standard deviation of the weight of 30 individual dosage forms produced is less than 1%, preferably 0.5% and the dosage form does not have a free-formed surface.

Also described are dosage forms which comprise at least one active ingredient and a confectionery composition wherein the dosage form has at least one face, does not have a free formed surface, and has a mean polarized light transmission at the angle of maximum extinction which is not greater than the mean polarized light transmission of the dosage form at the angle of maximum transmission.

In another embodiment, the relative standard deviation of the weight of the dosage form is less than 0.5%.

In another embodiment, the confectionery composition does not contain a gelatin based composition.

In another embodiment, the confectionery composition does not contain a gel based composition.

The confectionery composition comprises an amorphous sugar glass component and the dosage form does not have a free formed surface.

In another embodiment, all the faces of the dosage form have a surface gloss of about 200-300 gloss units.

In another embodiment, the dosage form has two or more faces, and the difference in surface gloss between any two faces is not more than about 20 gloss units.

In another embodiment, the differences in surface gloss between any two faces is not more than about 15 gloss units.

In another embodiment, the differences in surface gloss between any two faces is not more than about 10 gloss units.

In another embodiment, the dosage form has a mean polarized light transmission at the angle of maximum extinction which is not greater than the mean polarized light transmission of the dosage form at the angle of maximum transmission.

In another embodiment, the dosage form has a mean polarized light transmission between about 0 to 40 grayscale units at the angle of maximum extinction.

In another embodiment, the confectionery composition comprises an amorphous sugar glass component and the dosage form does not have a free formed surface.

In another embodiment, the active ingredient is a pharmaceutically active ingredient.

In another embodiment, the dosage form comprises particles which comprise the active ingredient.

In another embodiment, the particles have an average particle size of about 50 to about 2000 microns.

In another embodiment, at least a portion of the particles are coated particles.

In another embodiment, the dosage form is a unitary object.

In another embodiment, the dosage form does not contain any seams on its surface.

In another embodiment, the relative standard deviation of the weight of the dosage form is less than 1.0%.

In another embodiment, the confectionery composition comprises an amorphous sugar glass component.

In another embodiment, the dosage form is substantially free of pores having a diameter of 0.5 to 5.0 microns.

In another embodiment, the dosage form of this invention does not contain a gel-based composition or a gelatin-based composition.

In another embodiment, the dosage form of this invention comprises an amorphous sugar glass component and the dosage form does not have a free formed surface.

In another embodiment, all the dosage form faces have a surface gloss of about 200-300 gloss units.

In another embodiment, the dosage form has two or more faces, and the difference in surface gloss between any two faces is not more than about 20 gloss units, preferably not more than about 15 gloss units, most preferably not more than about 10 gloss units.

In another embodiment, the dosage form has a mean polarized light transmission between about 0 to 40 grayscale units at the angle of maximum extinction.

In another embodiment, the active ingredient is a pharmaceutically active ingredient.

### BRIEF DESCRIPTION OF THE DRAWINGS

Figure 1A depicts a prior art composition and a composition of the present invention viewed under normal polarized light.

Figure 1B depicts a prior art composition and a composition of the present invention viewed under polarized light at 90° filtering to the incident light.

Figure 2A is a photograph of a two-piece hard candy lozenge mold which may be used to prepare the dosage form of this invention.

Figure 2B is a photograph of the mold depicted in Fig. 2A together with a lozenge dosage form which has been removed from the mold.

Figure 3A depicts an apparatus for measuring mean polarized light transmission at the angle of maximum transmission.

Figure 3B depicts an apparatus for measuring mean polarized light transmission at the angle of maximum extinction.

Figure 4A depicts typical photographic images taken at the angle of maximum light transmission comparing hard candies made by methods of the prior art and the dosage form of this invention.

Figure 4B depicts typical photographic images taken at the angle of maximum light extinction comparing hard candies made by methods of the prior art and the dosage form of this invention.

Figure 5 shows the greyscale intensity distribution for a CHLORASEPTIC throat lozenge at both the angle of maximum light transmission and the angle of maximum light extinction.

Figure 6 shows the greyscale intensity distribution for the product of the present invention at both the angle of maximum light transmission and the angle of maximum light extinction.

### DETAILED DESCRIPTION OF THE INVENTION

As used herein, the term "dosage form" applies to any solid object, semi-solid, or liquid composition, designed to contain a specific pre-determined amount (i.e. dose) of a certain ingredient, for example an active ingredient as defined below. Suitable dosage forms may be pharmaceutical drug delivery systems, including those for oral administration, buccal administration, rectal administration, topical, transdermal, or mucosal delivery, or subcutaneous implants, or other implanted drug delivery systems; or compositions for delivering minerals, vitamins and other nutraceuticals, oral care agents, flavorants, and the like. Preferably the dosage forms of the present invention are considered to be solid, however they may contain liquid or semi-solid components. In a particularly preferred embodiment, the dosage form is an orally administered system for delivering a pharmaceutical active ingredient to the gastro-intestinal tract of a human. In another preferred embodiment, the dosage form is an orally administered "placebo" system containing pharmaceutically inactive ingredients, and the dosage form is designed to have the same appearance as a particular pharmaceutically active dosage form, such as may be used for control purposes in clinical studies to test, for example, the safety and efficacy of a particular pharmaceutically active ingredient.

The dosage form of this invention must be molded. The dosage form comprises at least one active ingredient and a confectionery composition.

Suitable active ingredients for use in this invention include for example pharmaceuticals, minerals, vitamins and other nutraceuticals, oral care agents, flavorants and mixtures thereof. Suitable pharmaceuticals include analgesics, anti-inflammatory agents, antiarthritics, anesthetics, antihistamines, antitussives, antibiotics, anti-infective agents, antivirals, anticoagulants, antidepressants, antidiabetic agents, antiemetics, antiflatulents, antifungals, antispasmodics, appetite suppressants, bronchodilators, cardiovascular agents, central nervous system agents, central nervous system stimulants, decongestants, diuretics, expectorants, gastrointestinal agents, migraine preparations, motion sickness products, mucolytics, muscle relaxants, osteoporosis preparations, polydimethylsiloxanes, respiratory agents, sleep-aids, urinary tract agents and mixtures thereof.

Suitable oral care agents include breath fresheners, tooth whiteners, antimicrobial agents, tooth mineralizers, tooth decay inhibitors, topical anesthetics, mucoprotectants, and the like.

Suitable flavorants include menthol, peppermint, mint flavors, fruit flavors, chocolate, vanilla, bubble gum flavors, coffee flavors, liqueur flavors and combinations and the like.

Examples of suitable gastrointestinal agents include antacids such as calcium carbonate, magnesium hydroxide, magnesium oxide, magnesium carbonate, aluminum hydroxide, sodium bicarbonate, dihydroxyaluminum sodium carbonate; stimulant laxatives, such as bisacodyl, cascara sagrada, danthron, senna, phenolphthalein, aloe, castor oil, ricinoleic acid, and dehydrocholic acid, and mixtures thereof; H2 receptor antagonists, such as famotadine, ranitidine, cimetadine, nizatidine; proton pump inhibitors such as omeprazole or lansoprazole; gastrointestinal cytoprotectives, such as sucraflate and misoprostol; gastrointestinal prokinetics, such as prucalopride, antibiotics for H. pylori, such as clarithromycin, amoxicillin, tetracycline, and metronidazole; antidiarrheals, such as diphenoxylate and loperamide; glycopyrrolate; antiemetics, such as ondansetron, analgesics, such as mesalamine.

In one embodiment of the invention, the active agent may be selected from bisacodyl, famotadine, ranitidine, cimetidine, prucalopride, diphenoxylate, loperamide, lactase, mesalamine, bismuth, antacids, and pharmaceutically acceptable salts, esters, isomers, and mixtures thereof.

In another embodiment, the active agent is selected from analgesics, antiinflammatories, and antipyretics: e.g. non-steroidal anti-inflammatory drugs (NSAIDs), including propionic acid derivatives: e.g. ibuprofen, naproxen, ketoprofen and the like; acetic acid derivatives: e.g. indomethacin, diclofenac, sulindac, tolmetin, and the like; fenamic acid derivatives: e.g. mefanamic acid, meclofenamic acid, flufenamic acid, and the like; biphenylcarbodylic acid derivatives: e.g. diflunisal, flufenisal, and the like; and oxicams: e.g. piroxicam, sudoxicam, isoxicam, meloxicam, and the like. In a particularly preferred embodiment, the active agent is selected from propionic acid derivative NSAID: e.g. ibuprofen, naproxen, flurbiprofen, fenbufen, fenoprofen, indoprofen, ketoprofen, fluprofen, pirprofen, carprofen, oxaprozin, pranoprofen, suprofen, and pharmaceutically acceptable salts, derivatives, and combinations thereof. In another embodiment of the invention, the active agent may be selected from acetaminophen, acetyl salicylic acid, ibuprofen, naproxen, ketoprofen, flurbiprofen, diclofenac, cyclobenzaprine, meloxicam, rofecoxib, celecoxib, and pharmaceutically acceptable salts, esters, isomers, and mixtures thereof. In another embodiment of the invention, the active agent may be selected from acetaminophen, acetyl salicylic acid, ibuprofen, naproxen, ketoprofen, flurbiprofen, diclofenac, cyclobenzaprine, meloxicam, rofecoxib, celecoxib, and pharmaceutically acceptable salts, esters, isomers, and mixtures thereof.

In another embodiment of the invention, the active agent may be selected from pseudoephedrine, phenylpropanolamine, chlorpheniramine, dextromethorphan, diphenhydramine, doxylamine, astemizole, terfenadine, fexofenadine, loratadine, desloratadine, cetirizine, mixtures thereof and pharmaceutically acceptable salts, esters, isomers, and mixtures thereof.

Examples of suitable polydimethylsiloxanes, which include, but are not limited to dimethicone and simethicone, are those disclosed in United States Patent Nos. 4,906,478, 5,275,822, and 6,103,260. As used herein, the term "simethicone" refers to the broader class of polydimethylsiloxanes, including but not limited to simethicone and dimethicone.

The active ingredient or ingredients are present in the dosage form in a therapeutically effective amount, which is an amount that produces the desired therapeutic response upon oral administration and can be readily determined by one skilled in the art. In determining such amounts, the particular active ingredient being administered, the bioavailability characteristics of the active ingredient, the dose regime, the age and weight of the patient, and other factors must be considered, as known in the art. Preferably, the dosage form comprises at least about 85 weight percent of the active ingredient. In one preferred embodiment, the core comprises at least about 85 weight percent of the active ingredient.

The active ingredient or ingredients may be present in the dosage form in any form. For example, the active ingredient may be dispersed at the molecular level, e.g. melted or dissolved, within the dosage form, or may be in the form of particles, which in turn may be coated or uncoated. If the active ingredient is in form of particles, the particles (whether coated or uncoated) typically have an average particle size of about 1-2000 microns. In one preferred embodiment, such particles are crystals having an average particle size of about 1-300 microns. In another preferred embodiment, the particles are granules or pellets having an average particle size of about 50-2000 microns, preferably about 50-1000 microns, most preferably about 100-800 microns.

If the active ingredient has an objectionable taste, and the dosage form is intended to be chewed or disintegrated in the mouth prior to swallowing, the active ingredient may be coated with a taste masking coating, as known in the art. Examples of suitable taste masking coatings are described in U.S. Patent No. 4,851,226, U.S. Patent No. 5,075,114, and U.S. Patent No. 5,489,436. Commercially available taste masked active ingredients may also be employed. For example, acetaminophen particles which are encapsulated with ethylcellulose or other polymers by a coaccervation process may be used in the present invention. Coaccervation-encapsulated acetaminophen may be purchased commercially from Eurand America, Inc. (Vandalia, Ohio) or from Circa Inc. (Dayton, Ohio).

The dosage form of the invention may also incorporate pharmaceutically acceptable adjuvants, including, for example, preservatives, high intensity sweeteners such as aspartame, acesulfame potassium, cyclamate, saccharin, sucralose, and the like; and other sweeteners such as dihydroalcones, glycyrrhizin, Monellin™, stevioside, Talin™, and the like; flavors, antioxidants, surfactants, and coloring agents.

In certain embodiments in which modified release of the active ingredient is desired, the active ingredient may optionally be coated with a release-modifying coating, as is well known in the art. Commercially available modified release active ingredients may also be employed. For example, acetaminophen particles which are encapsulated with release-modifying polymers by a coaccervation process may be used in the present invention as described above.

The active ingredient or ingredients are preferably capable of dissolution upon contact with a fluid such as water, stomach acid, intestinal fluid or the like. In a preferred embodiment the dissolution characteristics of the active ingredient meet USP specifications for immediate release tablets containing the active ingredient. In embodiments in which it is desired for the active ingredient to be absorbed into the systemic circulation of an animal, the active ingredient or ingredients are preferably capable of dissolution upon contact with a fluid such as water, stomach acid, intestinal fluid or the like. In one embodiment, the dissolution characteristics of the active ingredient meet USP specifications for immediate release tablets containing the active ingredient. For example, for acetaminophen tablets, USP 24 specifies that in pH 5.8 phosphate buffer, using USP apparatus 2 (paddles) at 50 rpm, at least 80% of the acetaminophen contained in the dosage form is released therefrom within 30 minutes after dosing, and for ibuprofen tablets, USP 24 specifies that in pH 7.2 phosphate buffer, using USP apparatus 2 (paddles) at 50 rpm, at least 80% of the ibuprofen contained in the dosage form is released therefrom within 60 minutes after dosing. See USP 24, 2000 Version, 19-20 and 856 (1999). In another embodiment, the dissolution characteristics of the active ingredient are modified: e.g. controlled, sustained, extended, retarded, prolonged, delayed and the like.

As used herein, the term "confectionery composition" means an edible product comprising a sweet component. Confectionery compositions generally include medicinal preparations made with sugar, syrup, or honey, and sweet foods such as candy or pastry. Suitable confectionery compositions are well known in the art and include "sugar confectionery" such as hard candy (e.g. amorphous sugar-glass, toffees, fudge, fondants, jellies, gummis, pasteils, caramel, taffy, nougat, and chewing gum) as well as "fat-based confectionery" such as chocolate (including, for example, milk chocolate, dark chocolate, semi-sweet chocolate), and coatings including, for example, chocolate compound coatings, pastel compound coatings e.g. white chocolate, and the like.

Suitable sweet components include sugars such as sucrose, glucose (dextrose), fructose; sugar-alcohols such as sorbitol, mannitol, erythritol, xylitol, maltitol and the like; and high-intensity sweeteners such as aspartame, acesulfame potassium, cyclamate saccharin, sucralose, and the like; and other sweeteners such as dihydroalcones, glycyrrhizin, Monellin™, stevioside, Talin™, and the like.

In another embodiment, the confectionery composition may be selected from fat-based confectionery compositions, such as chocolate, including, for example, milk chocolate, dark chocolate, semi-sweet chocolate; coatings including, for example, chocolate compound coatings, pastel compound coatings e.g. white chocolate, and the like. In such embodiments, the fat-based confectionery composition may typically begin to melt at a temperature of about 25 to about 80°C, e.g. about 25 to about 40°C, or about 34 to about 37°C, or about 40 to about 80°C, and comprises a suitable low-melting material. Those skilled in the art will recognize that the melting point of the composition may vary according to the solid-fat indices of the various fat components. Solid-fat index (SFI) is a method of characterizing fat blends related to proportion of liquid fat in the blend at different temperatures. IUPAC method 2.141 "Determination of the Dilatation of Fats" details the method for measuring this index.

In embodiments where the confectionery composition begins to melt between 25-40°C, suitable low-melting materials include triglycerides such as lauric and non-lauric cocoa butter substitutes, lauric (shorter chain fatty acids) fats, such as coconut and palm kernal oils and derivatives thereof; non-lauric (longer chain fatty acids) fats such as cocoa butter, palm oil, soybean and cottonseed oils, and derivatives thereof; cocoa butter equivalents; mono, and diglycerides; phospholipids; and water soluble polymers such as polyethylene glycol. In one embodiment, the low-melting material is a fractionated lauric fat selected from palm kemal oil and coconut oil.

In embodiments where the composition begins to melt between 40 - 80°C, suitable low-melting materials include waxes such as carnauba wax, spermaceti wax, beeswax, candelilla wax, shellac wax, microcrystalline wax, and paraffin wax, water soluble polymers such as polyethylene glycol, polyethylene oxides and derivatives, and sucrose esters.

In yet another embodiment, the confectionery composition may be a fondant. In such embodiments, the confectionery composition comprises a crystalline carbohydrate of which at least about 90% of the crystals have an average size from about 2 to about 15 microns, and typically has a moisture content of about 5 to about 15%, e.g. about 10 to about 12%.

In a preferred embodiment, the confectionery composition comprises at least one component selected from fat, amorphous sugar glass and fondant.

The dosage form of this invention may have any shape that is suitable for molding. For example, in one embodiment the dosage form may be in the shape of a truncated cone. In other embodiments the dosage form may be shaped as a polyhedron, such as a cube, pyramid, prism, or the like; or may have the geometry of a space figure with some non-flat faces, such as a cone, cylinder, sphere; torus, or the like. Exemplary shapes which may be employed include tablet shapes formed from compression tooling shapes described by "The Elizabeth Companies Tablet Design Training Manual" (Elizabeth Carbide Die Co., Inc., p.7 (McKeesport, Pa.) (incorporated herein by reference) as follows (the tablet shape corresponds inversely to the shape of the compression tooling):
1. Shallow Concave.
2. Standard Concave.
3. Deep Concave.
4. Extra Deep Concave.
5. Modified Ball Concave.
6. Standard Concave Bisect.
7. Standard Concave Double Bisect.
8. Standard Concave European Bisect.
9. Standard Concave Partial Bisect.
10. Double Radius.
11. Bevel & Concave.
12. Flat Plain.
13. Flat-Faced-Beveled Edge (F.F.B.E.).
14. F.F.B.E. Bisect.
15. F.F.B.E. Double Bisect.
16. Ring.
17. Dimple.
18. Ellipse.
19. Oval.
20. Capsule.
21. Rectangle.
22. Square.
23. Triangle.
24. Hexagon.
25. Pentagon.
26. Octagon.
27. Diamond.
28. Arrowhead.
29. Bullet.
30. Barrel.
31. Half Moon.
32. Shield.
33. Heart.
34. Almond.
35. House/Home Plate.
36. Parallelogram.
37. Trapezoid.
38. Figure 8/Bar Bell.
39. Bow Tie.
40. Uneven Triangle.

The dosage form surface may be substantially smooth, i.e. may have indentations or protrusions at the microscopic level on the order of less than about 20 microns in width, depth, or height. Alternately the dosage form surface may be textured, i.e. having indentations or protrusions greater than about 20 microns, e.g. greater than about 50 microns, or greater than about 100 microns, say greater than about 1000 microns in width, depth, or height. The indentations or protrusions may be up to about 30,000 microns, e.g. up to about 2,000 microns in width, depth, or height. In embodiments wherein the dosage form surface is textured, the outer surface of the dosage form may contain an embossed (raised) or debossed (indented) design. For example, the outer surface of the core may contain indentations, intagliations, letters, symbols or a pattern such as a graphic or logo.
The dosage form has at least one face, and the relative standard deviation of the weight of 30 individual dosage forms as produced by injecting at least one active ingredient and an amorphous sugar glass component into a molding chamber and molding into a desired shape using a thermal setting molding method and/or a thermal cycle molding method is less than 1%, preferably less than 0.5%. As used herein, relative standard deviation of the weight of the dosage form may be determined as follows: The weights of 30 individual dosage forms are determined to the nearest 0.1 milligram using an analytical balance. From the 30 individual weights for each sample, a mean, sample standard deviation, and relative standard deviation (% RSD) are calculated, as set forth in Example 5 herein. The relative standard deviation is the standard deviation expressed as a percentage of the mean, as is well known in the art.

The dosage form has at least one face, the relative standard deviation of the weight of the dosage form as defined herein is less than 1%, preferably less than 0.5%, the dosage form does not have a free formed surface, and comprises an amorphous glass sugar component.

In another embodiment of this invention, the dosage form has at least one face, and the relative standard deviation of the weight of the dosage form as defined herein is less than 1%, preferably less than 0.5%, and all the faces of the dosage form have a surface gloss of about 200-300 gloss units. As used herein, "surface gloss" means a measure of reflected light determined according to the method set forth in Example 4 herein.

In another embodiment of this invention, the dosage form has more than one face, the relative standard deviation of the weight of the dosage form as defined herein is less than 1%, preferably less than 0.5%, and the difference in surface gloss between any two faces is not more than about 20 gloss units, preferably about 15 gloss units, more preferably about 10 gloss units.

In another embodiment of this invention, the dosage form has at least one face, the relative standard deviation of the weight of the dosage form as defined herein is less than 1%, preferably less than 0.5%, and the dosage form has a mean polarized light transmission at the angle of maximum extinction which is not greater than the mean polarized light transmission of the dosage form at the angle of maximum transmission.

"Mean polarized light-transmission" as used herein is measured according the method set forth in Example 6 herein. In summary, the system for measuring the mean polarized light transmission consists of a light source followed by a first polarizing filter, followed by the sample, followed by a second polarizing filter, followed by a detector, such as, for example, a digital camera.

The "angle of maximum transmission" as used herein is the relative position of the polarizers that allows the maximum amount of light to pass through the sample. This has been found to be an angle of approximately zero degrees between the first and second polarizers.

The "angle of maximum extinction" as used herein is the relative position of the polarizers that allows the minimum amount of light to pass through a sample. This has been found to be an angle between the first and second polarizers of approximately 90 degrees, plus or minus about 20 degrees. The angle of maximum extinction will vary with the composition of the sample because different sugar-containing compositions rotate the plane of polarized light by varying amounts, with the average being about minus 15 degrees for a 100% glucose solution which corresponds to an angle of maximum extinction of about 75 degrees between the first and second polarizers.

The polarized light transmission is measured by plotting the intensity of transmitted light across the 256 grayscale values of the detected image. The mean polarized light transmission is defined as the grayscale value at the midpoint of the area of peak transmission intensity. The difference between the mean polarized light transmission at the angle of maximum transmission and the mean polarized light transmission at the angle of maximum extinction is referred to herein as the "relative homogeneity index." A sample which is free of strain lines and air bubbles will have a high relative homogeneity index, while a sample containing strain lines and air bubbles will have a low relative homogeneity index.

In another embodiment of this invention, the dosage form has at least one face, the relative standard deviation of the weight of the dosage form as described herein is less than 1%, preferably less than 0.5%, the dosage form has a mean polarized light transmission at the angle of maximum extinction which is not greater than the mean polarized light transmission of the dosage form at the angle of maximum transmission, and the dosage form has a mean polarized light transmission between about 0 to 40 grayscale units at the angle of maximum. extinction.

Also described are dosage forms having at least one face, which do not have a free formed surface, and which have a mean polarized light transmission at the angle of maximum extinction which is not greater than the mean polarized light transmission of the dosage form at the angle of maximum transmission.

In another embodiment of this invention, the dosage form has at least one face, does not have a free formed surface, and has a mean polarized light transmission at the angle of maximum extinction which is not greater than the mean polarized light transmission of the dosage form at the angle of maximum transmission, and the relative standard deviation of the weight of the dosage form as defined herein is less than 1.0%, preferably less than 0.5%, more preferably less than 0.5%.

In another embodiment, the dosage form has at least one face, does not have a free formed surface, and has a mean polarized light transmission at the angle of maximum extinction which is not greater than the mean polarized light transmission of the dosage form at the angle of maximum transmission, and the confectionery composition does not contain a gelatin based or gel based composition.

In another embodiment, the dosage form has at least one face, does not have a free formed surface, and has a mean polarized light transmission at the angle of maximum extinction which is not greater than the mean polarized light transmission of the dosage form at the angle of maximum transmission and the confectionery composition comprises an amorphous sugar glass component.

In another embodiment, the dosage form has two or more faces, does not have a free formed surface, and has a mean polarized light transmission at the angle of maximum extinction which is not greater than the mean polarized light transmission of the dosage form at the angle of maximum transmission and all of the faces of the dosage form have a surface gloss of about 200-300 gloss units.

In another embodiment, the dosage form has two or more faces, does not have a free formed surface, and has a mean polarized light transmission at the angle of maximum extinction which is not greater than the mean polarized light transmission of the dosage form at the angle of maximum transmission, and the difference in surface gloss between any two faces is not more than about 20 gloss units, preferably about 15 gloss units, most preferably about 10 gloss units.

The dosage form comprises a confectionery composition which is an amorphous sugar-glass. In such embodiments the dosage form has a mean polarized light transmission at the angle of maximum extinction which is not greater than the mean polarized light transmission of the dosage form at the angle of maximum transmission.

In another embodiment, the dosage form of this invention is a unitary object, that is, a single, undivided article or product similar to a piece of hard candy, for example.

In another preferred embodiment, the dosage form of this invention does not contain any seams on its surface.

In other embodiments, the dosage form of this invention is substantially free of pores having a diameter of 0.5 to 5.0 microns. By "substantially free" it is meant that the dosage form has a pore volume of less than about 0.02 cc/g, preferably less than about 0.01 cc/g, more preferably less than about 0.005 cc/g, in the pore diameter range of 0.5 to 5.0 microns. Typical compressed materials have pore volumes of more than about 0.02 cc/g in this pore diameter range.

Pore volume measurements may be obtained using a Quantachrome Instruments PoreMaster 60 mercury intrusion porosimeter and associated computer software program known as "Porowin." The procedure is documented in the Quantachrome Instruments PoreMaster Operation Manual. The PoreMaster determines both pore volume and pore diameter of a solid or powder by forced intrusion of a non-wetting liquid (mercury), which involves evacuation of the sample in a sample cell (penetrometer), filling the cell with mercury to surround the sample with mercury, applying pressure to the sample cell by: (i) compressed air (up to 50 psi maximum); and (ii) a hydraulic (oil) pressure generator (up to 60000 psi maximum). Intruded volume is measured by a change in the capacitance as mercury moves from outside the sample into its pores under applied pressure. The corresponding pore size diameter (d) at which the intrusion takes place is calculated directly from the so-called "Washburn Equation": d= -(4γ(cosθ))/P where y is the surface tension of liquid mercury, θ is the contact angle between mercury and the sample surface and P is the applied pressure.

### Equipment used for pore volume measurements:

1. Quantachrome Instruments PoreMaster 60.
2. Analytical Balance capable of weighing to 0.0001g.
3. Desiccator.

### Reagents used for measurements:

1. High purity nitrogen.
2. Triply distilled mercury.
3. High pressure fluid (Dila AX, available from Shell Chemical Co.).
4. Liquid nitrogen (for Hg vapor cold trap).
5. Isopropanol or methanol for cleaning sample cells.
6. Liquid detergent for cell cleaning.

### Procedure:

The samples remain in sealed packages or as received in the dessicator until analysis. The vacuum pump is switched on, the mercury vapor cold trap is filled with liquid nitrogen, the compressed gas supply is regulated at 55 psi., and the instrument is turned on and allowed a warm up time of at least 30 minutes. The empty penetrometer cell is assembled as described in the instrument manual and its weight is recorded. The cell is installed in the low pressure station and "evacuation and fill only" is selected from the analysis menu, and the following settings are employed:
Fine Evacuation time: 1 min.
Fine Evacuation rate: 10
Coarse Evacuation time: 5 min.

The cell (filled with mercury) is then removed and weighed. The cell is then emptied into the mercury reservoir, and two tablets from each sample are placed in the cell and the cell is reassembled. The weight of the cell and sample are then recorded. The cell is then installed in the low-pressure station, the low-pressure option is selected from the menu, and the following parameters are set:
Mode: Low pressure
Fine evacuation rate: 10
Fine evacuation until: 2001µ Hg
Coarse evacuation time: 10 min.
Fill pressure: Contact +0.1
Maximum pressure: 50
Direction: Intrusion And Extrusion
Repeat: 0
Mercury contact angle; 140
Mercury surface tension: 480

Data acquisition is then begun. The pressure vs. cumulative volume-intruded plot is displayed on the screen. After low-pressure analysis is complete, the cell is removed from the low-pressure station and reweighed. The space above the mercury is filled with hydraulic oil, and the cell is assembled and installed in the high-pressure cavity. The following settings are used:
Mode: Fixed rate
Motor speed: 5
Start pressure: 20
End pressure: 60 000
Direction: Intrusion and extrusion
Repeat: 0
Oil fill length: 5
Mercury contact angle: 140
Mercury surface tension: 480

Data acquisition is then begun and graphic plot pressure vs. intruded volume is displayed on the screen. After the high pressure run is complete, the low-and high-pressure data files of the same sample are merged.

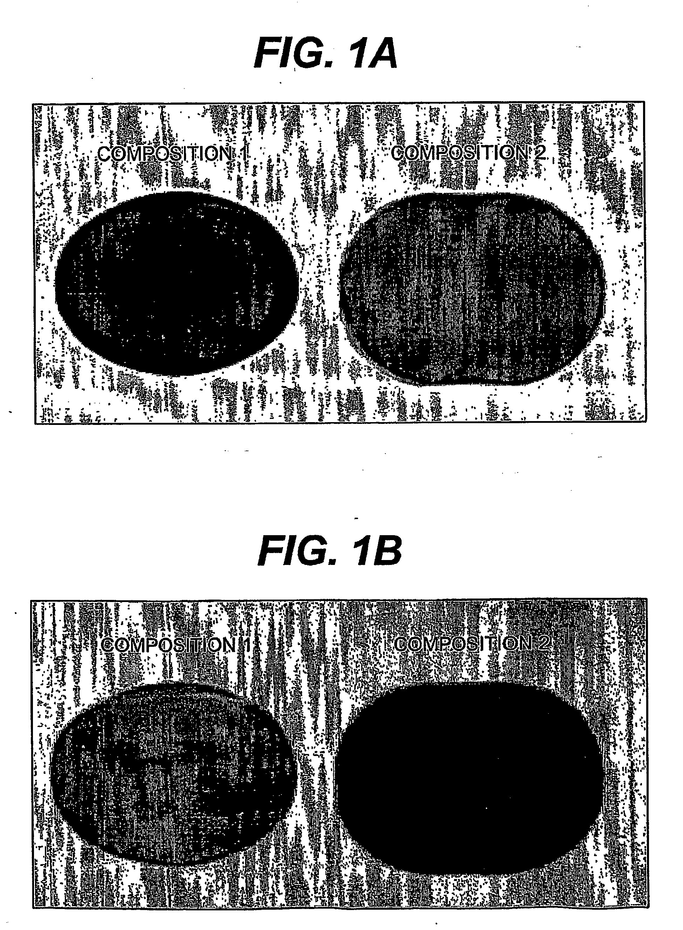

The differences between the prior art and the present invention will be further understood with reference to Figures 1A and 1B. In Figure 1A, Composition 1 is a Halls hard candy composition commercially available from Warner-Lambert Co., Morris Plains, NJ, and Composition 2 is dosage form according to the invention in which the confectionery composition is amorphous sugar glass. As show in Fig. 1A, when viewed under normal polarized light there are no distinguishing differences between Compositions 1 and 2. However, as shown in Fig. 1B, when viewed under polarized light at 90° filtering to the incident light, Composition 1 exhibits birefringence (i.e., refracted light) due to residual strain in the glass portion of the composition, whereas Composition 2 advantageously appears uniform without residual strain.
The dosage form of this invention is produced by molding, specifically, injection molding. The dosage form of the invention is produced by a thermal setting molding method and/or a thermal cycle molding method. It is preferred that the dosage form be made using the thermal setting molding method and apparatus described in copending U.S. Application Serial No. 09/966, 450, pages 57-63, or the thermal cycle molding method and apparatus described in copending U.S. Application Serial No. 09/966,497, pages 27-51.

In the thermal setting molding method, a starting material comprising the ingredients for the dosage form is injected into a molding chamber and molded into the desired shape. The starting material preferably comprises, in addition to the active ingredient and the confectionary composition, a thermal setting material at a temperature above the melting point of the thermal setting material but below the decomposition temperature of the active ingredient. The starting material is cooled and solidifies in the molding chamber into a shaped pellet (i.e., having the shape of the mold).

According to this method, the starting material must be in flowable form. For example, it may comprise solid particles suspended in a molten matrix, for example a polymer matrix. The starting material may be completely molten or in the form of a paste. The starting material may comprise an active ingredient dissolved in a molten material. Alternatively, the starting material may be made by dissolving a solid in a solvent, and the solvent is then evaporated from the starting material after it has been molded.

The thermal setting material may be any edible material that is flowable at a temperature between about 37 and about 250°C, and that is a solid or semi-solid at a temperature between about -10°C and about 35°C. In certain embodiments, the thermal setting material may comprise all or a portion of the confectionary composition as well, as some of the same materials are useful for both. Preferred thermal setting materials include water-soluble polymers such as polyalkylene glycols, polyethylene oxides and derivatives, and sucrose esters; fats such as cocoa butter, hydrogenated vegetable oil such as palm kernel oil, cottonseed oil, sunflower oil, and soybean oil; free fatty acids and their salts; mono- di-and triglycerides, phospholipids, waxes such as carnuba wax, spermaceti wax, beeswax, candelilla wax, shellac wax, microcrystalline wax, and paraffin wax; fat-containing mixtures such as chocolate; sugar in the form on an amorphous glass, sugar in a supersaturated solution,; low-moisture polymer solutions such as mixtures of gelatin and other hydrocolloids at water contents up to about 30% such as those used to make "gummi" confection forms. In a particularly preferred embodiment, the thermal setting material is a blend of fats and mono-and diglycerides.

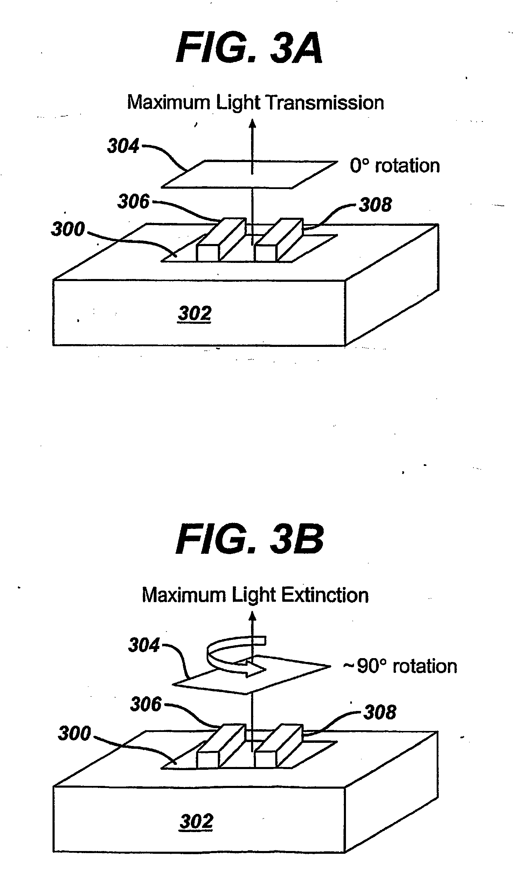

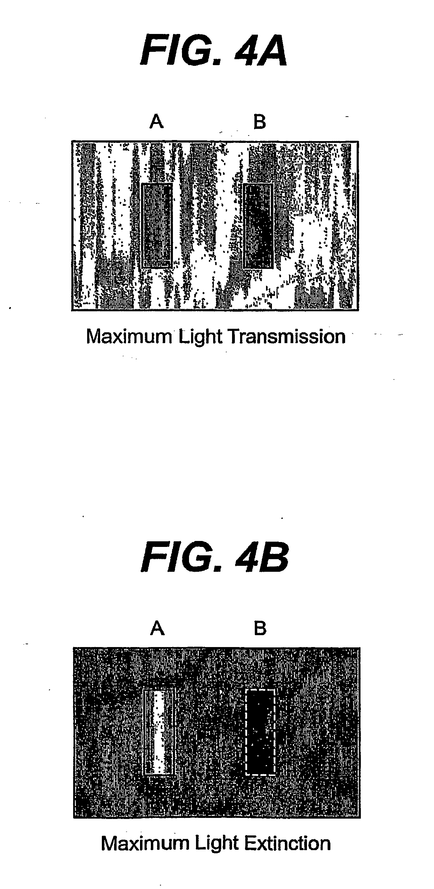

In the thermal cycle molding method and apparatus of U.S. patent application Serial No. 09/966,497, a thermal cycle molding module having the general configuration shown in Figure 3 therein is employed. The thermal cycle molding module 200 comprises a rotor 202 around which a plurality of mold units 204 are disposed. The thermal cycle molding module includes a reservoir 206 (see Figure 4) for holding flowable material to make the core. In addition, the thermal cycle molding module is provided with a temperature control system for rapidly heating and cooling the mold units. Figures 55 and 56 of the '497 application depict the temperature control system 600.

In this embodiment, the mold units preferably comprise center mold assemblies 212 and upper mold assemblies 214 as shown in Figure 26C of U.S. patent application Serial No. 09/966,497, which mate to form mold cavities having the desired shape of the dosage form. As rotor 202 rotates, the opposing center and upper mold assemblies close. Flowable material comprising the ingredients of the dosage form, which is heated to a flowable state in reservoir 206, is injected into the resulting mold cavities. The temperature of the flowable material is then decreased, hardening the flowable material into dosage forms. The mold assemblies open and eject the dosage forms.

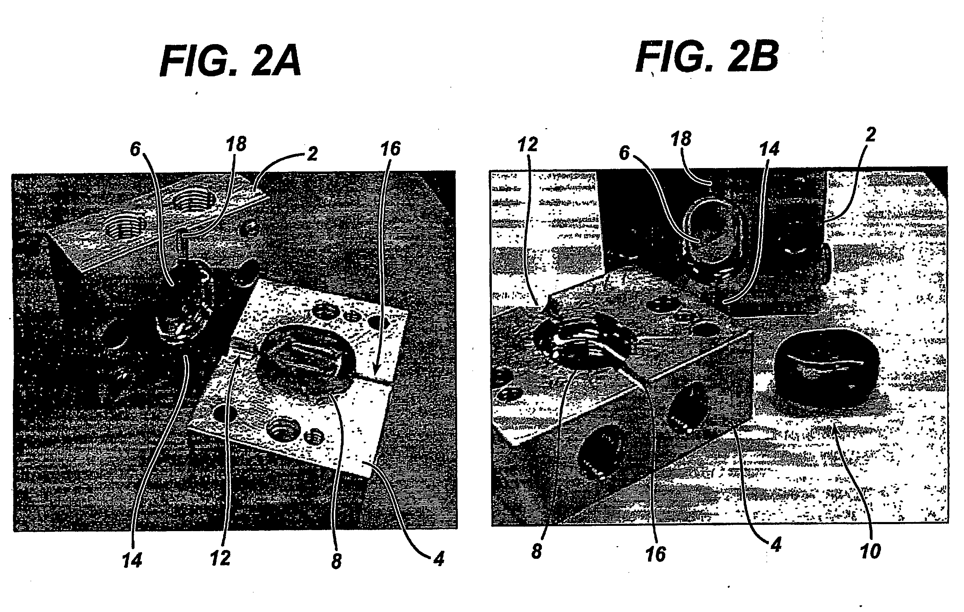

Fig. 2A is a photograph of a two-piece hard candy lozenge mold which may be used to prepare the dosage form of this invention. The mold in Figure 2A is a 2-piece aluminum mold made up of first mold piece 2 and second mold piece 4. Mold pieces 2 and 4 have respective cavity portions 6 and 8 which form a cavity which has been polished to a mirror finish. Mold pieces 2 and 4 also have respective injection port portions 12 and 14 which define an injection port for injecting flowable material into the mold, as well as vent port portions 16 and 18 for venting air. Fig. 2B is a photograph of the mold depicted in Fig. 2A together with a lozenge dosage form 10 which has been removed from the mold.

This invention will be further illustrated by the following examples.

### Example 1: Molded Hard Candy Dosage Form

A batch of molded hard candy lozenges was prepared in the following manner:

### Hard Candy Pre-cook Formulation:

The following ingredients were used to prepare the formulation:

| Ingredient | % | mg / tab | gms / batch |
|---|---|---|---|
| Corn Syrup (42 DE) | 45.35 | 170.06 | 498.85 |
| Sugar (dry granular) | 45.35 | 170.06 | 498.85 |
| Purified Water USP | 9.05 | 33.93 | 99.60 |
| FD&C Red #40 | 0.05 | 0.20 | 0.50 |
| Flavor | 0.20 | 0.75 | 2.20 |
| TOTAL | 100.00 | 375.00 | 1100.0 |

The corn syrup was weighed into a tared non-stick 2-quart saucepan. Purified water was then added to the same pan and the mixture was warmed over low heat with stirring until homogeneous. To the diluted corn syrup, dry granular sugar was added and stirred to form a wet slurry.

### Cook Step:

The sugar, water, corn syrup mixture was heated rapidly on a gas-fired stove over high heat with gentle stirring until the mixture appeared clear and began to boil. Heating continued until the mixture reached a temperature of 120° C at which point the heat was reduced to a medium flame. Heating was continued over a medium flame until 140°C was reached at which point the color was added to the batch and stirred in until uniformly distributed. When the temperature reached 145°C, the batch was removed from the stove and the flavor was blended into the batch. Subsequently, the batch was allowed to cool slightly without mixing so that air bubbles would dissipate.

### Molding Step:

While maintaining the batch in a fluid state by occasional heating, the molten candy base was injected into a two piece metal mold held together by thumbscrews (as shown in Figs. 2A and 2B). The mold, constructed from aluminum, had an injection port and a vent port that allowed excess candy mass to exit the mold. The fill cavity, in the shape of the desired lozenge piece, was polished to provide a molded dosage form having a smooth shiny surface. The injection device, a 10 cc plastic syringe having a cut off tip, was used to transfer candy base from the cooked batch and inject the candy base into the injection port of the molding apparatus. Once filled, the mold was allowed to cool at room temperature for about 2 minutes before opening. Upon opening, the molded dosage form was removed and any excess hard candy web from the fill and vent ports was snapped off.

### Example 2: Hard Candy Dosage Form

A dosage form according to the invention is made following the procedure stated in Example 1, except the hard candy pre-cook formation is as follows:

| Ingredient | % | mg / tab | gms / batch |
|---|---|---|---|
| Corn Syrup (42 DE) | 44.99 | 170.06 | 498.85 |
| Sugar (dry granular) | 44.99 | 170.06 | 498.85 |
| Purified Water USP | 8.98 | 33.93 | 99.60 |
| FD&C Red #40 | 0.05 | 0.20 | 0.50 |
| Flavor | 0.20 | 0.75 | 2.20 |
| Benzydamine | 0.79 | 3.00 | 8.80 |
| TOTAL | 100.00 | 378.00 | 1108.8 |

Here, the active ingredient, benzydamine, is added along with the flavor after the batch is removed from the stove.

### Example 3: Hard Candy Menthol Lozenge Dosage Form

A commercial scale batch of hard candy menthol dosage forms according to the invention is made using thermal cycle molding and the formulation set forth below:

### Hard Candy Pre-cook Formulation:

The following ingredients. including menthol as the active ingredient, are used to prepare the formulation:

| Ingredient | Supplier | Percent | Mg/tab | Theory Kg/batch |
|---|---|---|---|---|
| Sucrose (dry granular) | Florida Crystals, FL | 46.91 | 1407..3 | 234.6 |
| Corn Syrup 42 DE | Archer Daniel Midland, CA | 38.44 | 1153.2 | 192.2 |
| Menthol | A1 Menthol Century Int'l, CA | 0.40* | 12.0* | 2.00* |
| Purified Water | | 14.0 | 420.0 | 70.0 |
| Flavor | | 0.20 | 6.00 | 1.00 |
| Color | | 0.05 | 1.5 | 0.25 |
| TOTAL | | 100.0 | 3000 | 500.0 |

| | | | | |
|---|---|---|---|---|
| * Includes a 17% overage for loss during processing. | | | | |

Sucrose, corn syrup and water from the pre-cook formulation is vacuum cooked by a batch method. Other suitable methods include semicontinuous, and continuous vacuum cooking. Confectionery vacuum cookers are manufactured by APV Baker (Peterborough, UK), Kloeckner-Hansel GmbH (Cape Coral, FL), and Hosokawa Ter Braak B.V. (Rotterdam, Netherlands).

Sugar and water are added to the syrup cooking pan of the vacuum cooker and heated to about 110 °C to completely dissolve the sugar crystals. Subsequently, the corn syrup is added and the mixture is cooked to about 138°C and then discharged to the lower pan under vacuum (620 mm). In the lower pan, color, flavor, and menthol are added and mixed into the batch.

While still fluid, the cooked hard candy batch is transferred to heated supply tanks or reservoirs, such as those shown as 206 in Figure 4 of copending U.S. Application Serial No. 09/966,497. The cooked hard candy batch is maintained within the reservoirs at about 120°C without stirring. The reservoirs 206 are covered and pressurized to about 150 psi or sufficient pressure to allow the fluid hard candy mass to flow to a thermal cycle molding module as follows.

The dosage forms are then made in a thermal cycle molding module thermal cycle molding module having the specific configuration shown in Figure 26A of copending U.S. Application Serial No. 09/966,497. The thermal cycle molding module comprises center mold assemblies 212 and upper mold assemblies 214 as shown in Figure 26C, which mate to form mold cavities having the shape of a hard candy. As rotor 202 rotates, the opposing center and upper mold assemblies close. The fluid, cooked hard candy batch is injected into the resulting mold cavities.

The mold assemblies are thermally cycled between about 120 °C and about 90 °C and receive the hot cooked hard candy batch material when its cycle is in the high temperature range. As the hot material fills the mold cavity, the mold temperature is cycled to the low temperature range (i.e. 60 - 90°C) and cools the material to a temperature where it is sufficiently solid that it no longer flows under its own weight. Once set (i.e., for 1-60 seconds), the mold assemblies open and the molded dosage form is ejected from the thermal cycle molding module and conveyed to a tray to cool and completely harden.

Once cooled to room temperature (22°C), a batch of molded hard candy lozenge dosage forms is ready to be transferred in bulk to a packaging line.

### Example 4: Surface Gloss Comparisons

Commercially available molded lozenges were tested for surface gloss using an instrument available from TriCor Systems Inc. (Elgin, IL) under the tradename, " TRICOR MODEL 805A/806H SURFACE ANALYSIS SYSTEM" and generally in accordance with the procedure described in "TriCor Systems WGLOSS 3.4 Model 805A/806H Surface Analysis System Reference Manual" (1996), which is incorporated by reference herein, except as modified below,

This instrument utilized a CCD camera detector, employed a flat diffuse light source, compared tablet samples to a reference standard, and determined average gloss values at a 60 degree incident angle. During its operation, the instrument generated a gray-scale image, wherein the occurrence of brighter pixels indicated the presence of more gloss at that given location.

The instrument also incorporated software that utilized a grouping method to quantify gloss, i.e., pixels with similar brightness were grouped together for averaging purposes.

The "percent full scale" or "percent ideal" setting (also referred to as the "percent sample group" setting), was specified by the user to designate the portion of the brightest pixels above the threshold that will be considered as one group and averaged within that group. As used herein, "threshold" is defined as the maximum gloss value that will not be included in the average gloss value calculation. Thus, the background, or the non-glossy areas of a sample were excluded from the average gloss value calculations. The method disclosed in K. Fegley and C. Vesey, "The Effect of Tablet Shape on the Perception of High Gloss Film Coating Systems", which is available at www.colorcon.com as of 18 March, 2002 and incorporated by reference herein, was used to minimize the effects resulting from different dosage form shapes, and to report a metric that was comparable across the industry. (The 50% sample group setting was selected as the setting which best approximated analogous data from tablet surface roughness measurements.)

After initially calibrating the instrument using a calibration reference plate (190-228; 294 degree standard; no mask, rotation 0, depth 0), a standard surface gloss measurement was then created using gel-coated caplets available from McNEIL-PPC, Inc. under the tradename, "EXTRA STRENGTH TYLENOL GELCAPS." The average gloss value for a sample of 112 of such gel-coated caplets was then determined, while employing the 25 mm full view mask (190-280), and configuring the instrument to the following settings:
Rotation: 0
Depth: 0.25 inches
Gloss Threshold: 95
% Full Scale (% ideal): 50%
Index of Refraction: 1.57
The average surface gloss value for the reference standard was determined to be 269.

Each face of each sample molded lozenge was then independently tested in accordance with the same procedure. For the deposited samples, the "free-formed" face was designated as "Face 1." The opposing face was designated as "Face 2". A 31 by 31 pixel area (corresponding to an area on the sample of about 3.1 mm x 3.1 mm, or 9.61 square millimeters) was chosen to minimize contribution from air bubbles. HALLS PLUS lozenges available from Warner-Lambert Company, Morris Plains, NJ were selected as exemplary of lozenges manufactured by a conventional uniplasting process (Sample A). Cepacol® Sore Throat Lozenges, available from J.B. Williams Co., Glen Rock, NJ, and JOLLY RANCHER HARD CANDY available from Hershey Foods Corp, Hershey, PA were selected as exemplary of lozenges manufactured by conventional depositing methods (Samples B and C, respectively). Injection molded lozenges made according to the method of Example 1 were designated as Sample D. The results obtained were as follows:

| Sample | Manufacturing Method | Gloss - Face 1 | Gloss - Face 2 | Gloss - Difference (gloss units) |
|---|---|---|---|---|
| A (Halls Plus®) | Uniplast™ punch molded | 225 | 210 | 15 |
| B (Cepacol®) | Deposited | 282 | 261 | 21 |
| C (Jolly Rancher®) | Deposited | 296 | 256 | 40 |
| D (Example 1) | Injection Molded | 291 | 297 | 6 |

The results indicate that lozenge-type dosage forms according to the invention have a small difference in surface gloss between the two faces. In contrast, commercially available lozenges molded by depositing had surface glosses on their free-formed faces at least about 21 gloss units higher than the surface gloss on their molded faces. Lozenges made by the Uniplast punch method also showed less difference in surface gloss between their 2 major faces than those prepared by depositing.

### Example 5: Weight Variation of Comparative Products

Variation in piece weight of various commercially available confectionery forms, representative of conventional confectionery processing methods was measured by the following method. The weight of 30 individual pieces of each form was determined to the nearest 0.1 milligram using an analytical balance. From the 30 individual piece weights for each sample, a mean, sample standard deviation, and relative standard deviation (% RSD) were calculated. Note the relative standard deviation is the standard deviation expressed as a percentage of the mean, as known in the art.

| Product | Supplier | Mean Piece Weight (g) | Standard Deviation in piece weight (g) | Relative Standard Deviation in piece Weight (%) |
|---|---|---|---|---|
| Jolly Rancher® Hard Candy | Hershey Foods Corp, Hershey, PA | 5.9108 | 0.1972 | 3.34 |
| Tootsie Roll® | Tootsie Roll Industries, Inc, Chicago, IL | 6.5572 | 0.1248 | 1.90 |
| Hershey®'s Kisses® | Hershey Foods Corp, Hershey, PA | 4.6323 | 0.1523 | 3.29 |
| Halls® Cough Suppressant Drops | Warner-Lambert Company, Morris Plains, NJ | 3.7859 | 0.0652 | 1.72 |
| Werther®'s Original Hard Candy | Storck USA L.P., Chicago, IL | 5.1829 | 0.1294 | 2.50 |
| Kraft® Caramels | Nabisco Inc., East Hanover NJ | 7.9660 | 0.3844 | 4.83 |

### Example 6: Visualization and Quantification of Strain in Hard Candy Glass

Samples of hard candy glass were viewed and photographed under plane polarized light to quantify the relative strain in the candy glass. Sample A was a Halls Plus® lozenge commercially available from Warner- Lambert Co., Morris Plains, NJ. Sample B was an injection molded lozenge made according to Example 1 herein. Sample C was a Chloraseptic lozenge commercially available from Prestige Brands International, Inc., Bonita Springs, FL. Samples A and C are exemplary of Uniplas™ punch molded hard candy glass products. Typically, injection molded and deposited hard candy show no intrinsic strain whereas punch or roller molded hard candy retain residual strain that is visible when viewed under a strain viewer. The equipment used was as follows:
1) Camera - Scalar Portable Digital Microscope DG-1 fitted with the 1X lens with a C-mount adapter.
2) Strain Viewer - The strain viewer used was fabricated using:
   - Portable light box containing an 8 watt florescent lamp (Apollo - model LB101, Ronkonkoma, NY 11779).
   - Two 5"x 3" pieces of linear polarizing filters. Available from the 3M Company e.g., HN32 - Neutral linear polarizer or other suitable for stress analysis.

As depicted in Figs. 3A and 3B, a first filter 300 was secured to the middle of a light box 302 with cellophane tape and a second filter 304 was held by hand, parallel above the first filter 302. Samples (306 and 308) being analyzed were placed on top of the first filter 300 but beneath the second filter 304. Light transmission was regulated by rotating the second filter 304 while maintaining its parallel position above the first filter 300, as shown in Fig. 3B.

Alternatively, strain viewers are commercially available from: Strainoptic Technologies, Inc., 108 W. Montgomery Ave., North Wales, PA 19454, or from Sharples Stress Engineers, LTD, Unit 29 Old Mill Industrial Estate, School Lane, PRS 6SY Lancashire, UK.

Photographs of the samples were recorded using a digital camera. The pictures were taken through the second filter at both the maximum light transmission angle and the minimum light transmission angle (maximum extinction) with respect to the sample image (not the filter).

Figures 4A and 4B depict typical photographic images for a punch molded hard candy (Sample A) and an injection molded or deposited hard candy (Sample B) when viewed as described at maximum and minimum light transmission through a pair of polarizing filters.

Subsequently, image analysis software (Sigma Scan Pro 5 available from SPSS, Inc.) was used to quantitatively characterize the observed strain in the hard candy glass. Equivalent areas of the color images (free of bubble defects and cracks) were converted into gray scale and displayed as a histogram of pixel elements ranging in value from 0 (black) to 255 (white). For purposes of resolution, sample illumination and digital image exposure is adjusted to maximize the peak separation between the transmission and extinction states.

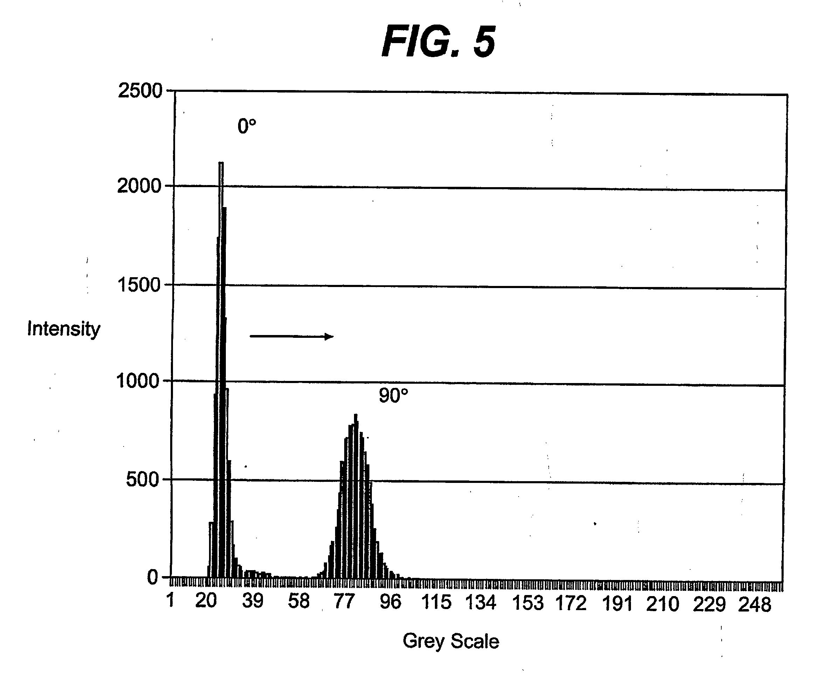

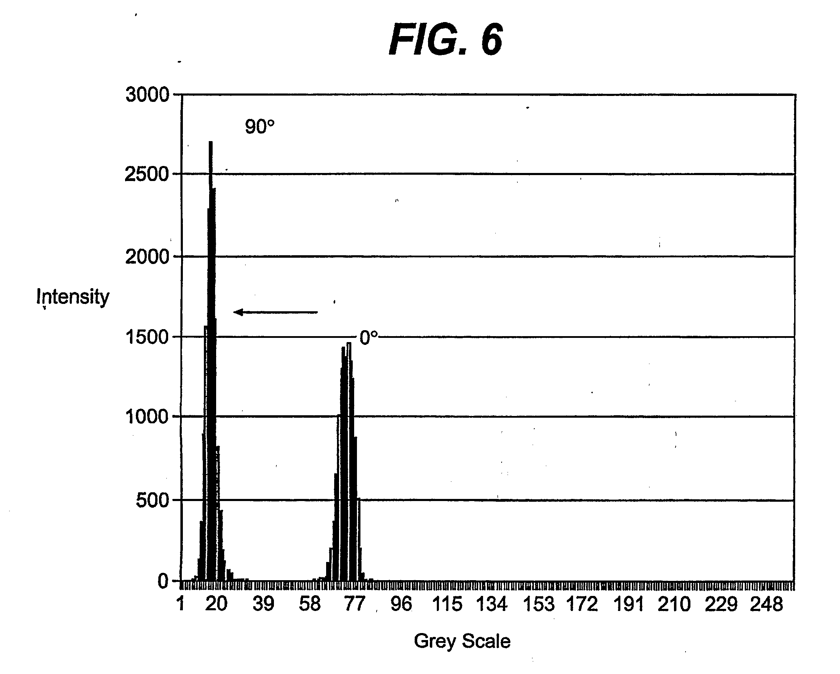

As seen in Figures 5 and 6, a converted image appears as a peak-like distribution of gray scale pixels. Moreover, the peak maximum for a given sample differs when the polarization filters are at maximum light transmission (0°) versus maximum light extinction (∼90°). Drop roll or punch molded hard candy samples, such as the Chloraceptic® Lozenge (Sample C) depicted in Figure 5, brighten as polarization reaches the point of maximum extinction and their gray scale peaks shift to higher values. Conversely, in the case of injection molded and deposited hard candy samples, their images darken and their gray scale peaks shift to smaller values as the polarized light reaches high extinction levels.

## Claims

1. A molded dosage form obtainable by injecting at least one active ingredient and an amorphous sugar glass component into a molding chamber and molding into a desired shape using a thermal setting molding method and/or a thermal cycle molding method wherein the relative standard deviation of the weight of 30 individual dosage forms produced is less than 1 %, the dosage form has at least one face and the dosage form does not have a free-formed surface.

2. The dosage form of Claim 1 in which dosage form has two or more faces, and the difference in surface gloss between any two faces is not more than 10 gloss units, wherein the surface gloss is measured in accordance with the procedure described in "Tricot Systems WGLOSS 4.3 Model 80SA/806H Surface Analysis System Reference Manual (1996)" employing the 25mm full view mask (190-280) at a 60° incident angle and configuring the instrument to the settings: Rotation 0, Depth 0.25 inches, Gloss threshold 95, % Full scale 50% and Index of Refraction: 1.57.

3. The dosage form of Claim 1, in which the dosage form has a mean polarized light transmission between 0 to 40 grayscale units at the angle of maximum extinction, wherein the mean polarized light transmission is measured using a camera and a strain viewer comprised of a portable light box containing an 8 watt fluorescent lamp and two pieces of linear polarizing filters wherein the two filters are arranged parallel to each other and the sample is placed on the top of the first filter but beneath the second.

4. The dosage form of Claim 1, in which the dosage form is a unitary object.

5. The dosage form of Claim 1, in which the dosage form does not contain any seams on its surface.

6. The dosage form of Claim 1, in which the dosage form is substantially free of pores having a diameter of 0.5 to 5.0 microns.

7. The dosage form of claim 1 in which the active ingredient and amorphous sugar glass component are molded using a thermal setting molding method.

8. The dosage form of claim 1, in which the active ingredient and amorphous sugar glass component are molded using a thermal cycle molding method.

## Patentansprüche

1. Geformte Dosierungsform, erhältlich durch Injizieren wenigstens eines Wirkstoffs und einer amorphen Zuckerglaskomponente in eine Formkammer und Formen zu einer gewünschten Gestalt unter Verwendung eines Wärmeerstarrungs-Formverfahrens und/oder eines Wärmezyklus-Formverfahrens, wobei die relative Standardabweichung des Gewichts von 30 einzelnen hergestellten Dosierungsformen weniger als 1 % beträgt, die Dosierungsform wenigstens eine Stirnseite aufweist und die Dosierungsform keine frei gebildete Oberfläche aufweist.

2. Dosierungsform gemäß Anspruch 1, welche Dosierungsform zwei oder mehr Stirnseiten aufweist und der Unterschied des Oberflächenglanzes zwischen beliebigen zwei Stirnseiten nicht mehr als 10 Glanzeinheiten beträgt, wobei der Oberflächenglanz gemäß dem in "Tricot Systems WGLOSS 4.3 Model 80SA/806H Surface Analysis System Reference Manual (1996)" beschriebenen Verfahren unter Verwendung der 25-mm-Full-View-Maske (190-280) mit einem Einfallwinkel von 60° und der Einstellung des Geräts mit den Konfiguration: Rotation 0, Tiefe 0,25 Inch, Glanzschwelle 95, % Endausschlag 50 % und Brechungsindex 1,57 gemessen wird.

3. Dosierungsform gemäß Anspruch 1, wobei die Dosierungsform eine mittlere Durchlässigkeit für polarisiertes Licht zwischen 0 und 40 Graustufeneinheiten bei dem Winkel maximaler Extinktion aufweist, wobei die mittlere Durchlässigkeit für polarisiertes Licht unter Verwendung einer Kamera und eines Spannungs-Viewers gemessen wird, der aus einer tragbaren Lichtkammer, die eine 8-Watt Fluoreszenzlampe enthält, und zwei Stück an linear polarisierenden Filtern zusammengesetzt ist, wobei die beiden Filter parallel zueinander angeordnet sind und die Probe auf dem ersten Filter aber unterhalb des zweiten angeordnet ist.

4. Dosierungsform gemäß Anspruch 1, wobei die Dosierungsform ein unitäres Objekt ist.

5. Dosierungsform gemäß Anspruch 1, wobei die Dosierungsform keine Falze an ihrer Oberfläche aufweist.

6. Dosierungsform gemäß Anspruch 1, wobei die Dosierungsform im Wesentlichen frei von Poren ist, die einen Durchmesser von 0,5 bis 5,0 Mikrometer aufweisen.

7. Dosierungsform gemäß Anspruch 1, wobei der Wirkstoff und die amorphe Zuckerglaskomponente unter Verwendung eines Wärmeerstarrungs-Formverfahrens geformt werden.

8. Dosierungsform gemäß Anspruch 1, wobei der Wirkstoff und die amorphe Zuckerglaskomponente unter Verwendung eines Wärmezyklus-Formverfahrens geformt werden.

## Revendications

1. Forme posologique moulée pouvant être obtenue en injectant au moins un ingrédient actif et un composant vitreux de sucre amorphe dans une chambre de moulage et en les moulant en une forme souhaitée en utilisant un procédé de moulage par fixage thermique et/ou un procédé de moulage à cycles thermiques dans lequel l'écart type relatif du poids de 30 formes posologiques individuelles produites est inférieur à 1 %, la forme posologique ayant au moins une face et la forme posologique n'ayant pas de surface formée librement.

2. Forme posologique selon la revendication 1, la forme posologique ayant au moins deux faces, et la différence de brillance de surface entre deux faces quelconques ne dépassant pas 10 unités de brillance, la brillance de surface étant mesurée conformément à la procédure décrite dans « Tricot Systems WGLOSS 4.3 Model 80SA/806H Surface Analysis System Reference Manual (1996) » en employant le masque panoramique de 25 mm (190-280) à un angle d'incidence de 60° et en configurant l'instrument aux réglages suivants : rotation 0, profondeur 0,25 pouce, seuil de brillance 95, % de la pleine échelle 50 %, et indice de réfraction 1,57.

3. Forme posologique selon la revendication 1, la forme posologique présentant une transmission moyenne de lumière polarisée entre 0 et 40 unités sur l'échelle des gris à l'angle d'extinction maximale, la transmission moyenne de lumière polarisée étant mesurée en utilisant une caméra et un polariscope composé d'un caisson lumineux portatif contenant une lampe fluorescente de 8 watts et deux morceaux de filtres polarisants linéaires, les deux filtres étant disposés parallèlement l'un à l'autre et l'échantillon étant placé au-dessus du premier filtre, mais en dessous du second.

4. Forme posologique selon la revendication 1, la forme posologique étant un objet unitaire.

5. Forme posologique selon la revendication 1, la forme posologique ne contenant aucune soudure sur sa surface.

6. Forme posologique selon la revendication 1, la forme posologique étant sensiblement dépourvue de pores ayant un diamètre de 0,5 à 5,0 µm.

7. Forme posologique selon la revendication 1, dans laquelle l'ingrédient actif et le composant vitreux de sucre amorphe sont moulés en utilisant un procédé de moulage par fixage thermique.

8. Forme posologique selon la revendication 1, dans laquelle l'ingrédient actif et le composant vitreux de sucre amorphe sont moulés en utilisant un procédé de moulage à cycles thermiques.
